# EUROPÄISCHE PATENTSCHRIFT

(11) **EP 1 956 896 B1**
(45) Veröffentlichungstag und Bekanntmachung des Hinweises auf die Patenterteilung: **10.08.2011**
(21) Anmeldenummer: 06818357.3
(22) Anmeldetag: 03.11.2006
(51) Int. Cl.: A01N 1/02, C12N 5/07

(54) **KRYOKONSERVIERUNG VON HEPATOCYTEN**
CRYOPRESERVATION OF HEPATOCYTES
CRYOCONSERVATION D'HEPATOCYTES

(30) Priorität: 25.11.2005 DE 102005057106
(43) Veröffentlichungstag der Anmeldung: 20.08.2008
(73) Patentinhaber: Cytonet GmbH & Co. KG, 69469 Weinheim (DE)
(72) Erfinder: ARSENIEV, Lubomir, 30625 Hannover (DE); ALEXANDROVA, Krassimira, 30625 Hannover (DE); BARTHOLD, Marc, 30171 Hannover (DE); KAFERT-KASTING, Sabine, 30173 Hannover (DE); LAUBE, Britta, 30539 Hannover (DE)
(74) Vertreter: Schwahn, Hartmut
(86) Internationale Anmeldenummer: PCT/EP2006/010549
(87) Internationale Veröffentlichungsnummer: WO 2007/059855

(56) Entgegenhaltungen:
- WO-A-97/38092
- KOEBE H G ET AL: "A NEW APPROACH TO THE CRYOPRESERVATION OF HEPATOCYTES IN A SANDWICH CULTURE CONFIGURATION" CRYOBIOLOGY, ACADEMIC PRESS INC, US, Bd. 27, Nr. 5, 1990, Seiten 576-584, XP009046283 ISSN: 0011-2240 in der Anmeldung erwähnt
- LLOYD TOM D R ET AL: "Cryopreservation of hepatocytes: a review of current methods for banking" CELL AND TISSUE BANKING, Bd. 4, Nr. 1, 2003, Seiten 3-15, XP002416013 ISSN: 1389-9333
- HUBEL A ET AL: "INTRACELLULAR ICE FORMATION DURING THE FREEZING OF HEPATOCYTES CULTURED IN A DOUBLE COLLAGEN GEL" BIOTECHNOLOGY PROGRESS, Bd. 7, Nr. 6, 1991, Seiten 554-559, XP002416014 ISSN: 8756-7938
- WATTS P ET AL: "CRYOPRESERVATION OF RAT HEPATOCYTE MONOLAYER CULTURES" HUMAN TOXICOLOGY, MACMILLAN PUBLISHERS, BASINGSTOKE, GB, Bd. 15, Nr. 1, 1996, Seiten 30-37, XP000926414 ISSN: 0144-5952 in der Anmeldung erwähnt
- MCKAY G C ET AL: "Cryopreservation of rat hepatocyte monolayers: Cell viability and cytochrome P450 content in post-thaw cultures" TOXICOLOGY IN VITRO, Bd. 16, Nr. 1, Februar 2002 (2002-02), Seiten 71-79, XP002416015 ISSN: 0887-2333
- STEVENSON D J ET AL: "Cryopreservation of viable hepatocyte monolayers in cryoprotectant media with high serum content: metabolism of testosterone and kaempherol post-cryopreservation" CRYOBIOLOGY, ACADEMIC PRESS INC, US, Bd. 49, Nr. 2, Oktober 2004 (2004-10), Seiten 97-113, XP004537698 ISSN: 0011-2240

## Beschreibung

Die vorliegende Anmeldung betrifft das technische Gebiet der Kryokonservierung und im Einzelnen Verfahren zur Präparation von Leberzellen für die Kryokonservierung, Verfahren zur Kryokonservierung isolierter Leberzellen und Verfahren zur Herstellung einer Sandwichkultur kryokonservierter isolierter Leberzellen.

Aus dem Gewerbeverband isolierte Leberzellen, speziell Hepatocyten, werden vor allem in Form von Primärzellkulturen zum Testen der physiologischen Wirkung von Arzneimittelkandidaten eingesetzt. Vor allem frisch präparierte primäre Hepatocyten aus Menschen stellen den "Goldstandard" dar, um in *in vitro*-Testreihen Wirkstoffkandidaten zu ermitteln oder Untersuchungen zum Metabolismus der Wirkstoffe oder zur Enzyminduktion durchzuführen. Nachteiligerweise sind frisch isolierte menschliche Hepatocyten nicht regelmäßig und jederzeit verfügbar. Es besteht daher der Bedarf an Verfahren, wodurch isolierte Hepatocyten für eine gewisse Zeit gelagert werden können. Die physiologische Funktion der Zellen, das heißt vor allem ihre metabolische beziehungsweise enzymatische Kompetenz, soll dabei möglichst vollständig erhalten bleiben.

Bekanntermaßen werden Hepatocyten kryokonserviert gelagert. In der Regel werden Hepatocyten in Suspension kryokonserviert. Die Suspension enthält neben den Hepatocyten noch ein Einfriermedium, das eine Schädigung der Zellen durch Einfrieren und Auftauen verhindern soll. Zur Lagerung wird die Suspension meist auf Temperaturen unterhalb von -80°C eingefroren. Zur Verwendung nach der Lagerung wird die gefrorene Zellsuspension aufgetaut, und die Zellen werden auf Kulturplatten oder in Kulturgefäße ausplattiert. Zur Rekultivierung der aufgebauten Zellen sind die Kulturgefäße in der Regel mit Matrixmaterial beschichtet, worauf die Zellen anhaften können. Eine erfolgreiche Anhaftung ist für die Rekultivierung und die nachfolgenden Untersuchungen essenziell. Meist lässt sich aber nur ein geringer Anteil der ursprünglich eingefrorenen Zellen in vitalem Zustand erhalten und rekultivieren. Der Nachteil dieses Vorgehens besteht vor allem darin, dass nicht vorhergesehen werden kann, ob oder zu welchem Anteil die aufgetauten Zellen aus der Suspension auf der Kulturplatte anhaften. Der Anteil der anhaftenden Zellen im Batch ist abhängig vom individuellen Batch. Regelmäßig heftet nur in einer geringen Zahl der Batches die aufgetauten kryokonservierten Zellen ausreichend an der Kulturplatte an.

Ein weiteres bekanntes Verfahren zur Kryokonservierung besteht darin, frisch isolierte Zellen in Kulturgefäßen auszuplattieren, um die ausplattierten Zellen anschließend zusammen mit den Kulturgefäßen einzufrieren. Auch hier werden die Kulturgefäße vor dem Ausplattieren mit einer Matrix, worauf die Zellen anhaften können, beschichtet. Bekanntermaßen werden bevorzugt Kollagengele eingesetzt. In einem bekannten Verfahren werden Hepatocyten auf mit Kollagen Typ I beschichteten Kulturplatten ausgesät und für cirka 4 Stunden auf der Kollagenmatrix anheften lassen. Die entstehende Monolayer-Kultur (Einschichtzellkultur) wird dann ungefähr 20 Stunden kultiviert. Anschließend werden nicht anhaftende Zellen abgewaschen. Nach weiteren 6 Stunden wird die Kultur mit Einfriermedium überschichtet, auf -70°C abgekühlt und gelagert (Watts und Grant, 1998; Human & Experimental Toxicology 15:30-37). Zur anschließenden Verwendung werden die Monolayerkulturen aufgetaut und rekultiviert. Die aufgetauten Kulturen weisen aber einen hohen Anteil nicht anhaftender und nicht vitaler Zellen sowie Zelltrümmer auf. Zwar sind meist große Teile der Matrix mit Zellen bedeckt, ein konfluenter, zusammenhängender Zellverband stellt sich jedoch nicht ein.

In einem anderen bekannten Verfahren werden Hepatocyten in Sandwichkonfiguration, das heißt in Doppelgel-Anordnung, eingefroren. Dazu wird eine Hepatocytensuspension zunächst auf mit Kollagengel beschichtete Zellkulturplatten ausgesät. Nach 24 Stunden Kultivierung der Zellen wird eine zweite Schicht aus Kollagengel auf die ausgesäten Zellen aufgegossen. Anschließend wird die so erhaltene Sandwichkultur in Einfriermedium auf -70°C eingefroren und gelagert (Koebe et al., 1990; Cryobiology 27:576-584).

Durch das Immobilisieren der Leberzellen auf einer Matrix oder zwischen zwei Matrices vor dem Einfrieren werden die Zellen mechanisch stabilisiert und dadurch die Überlebensrate erhöht. Dadurch, dass die Zellen in frisch isoliertem, hochvitalem Stadium anheften können, ist die Anheftungsrate gegenüber der Anheftung von in Suspension eingefrorener und aufgetauter kryokonservierter Zellen deutlich erhöht. Trotzdem treten auch hier nach dem Auftauen große Anteile nicht angehefteter beziehungsweise nicht vitaler Zellen auf. Der Idealzustand, eine konfluente Kultur vitaler Hepatocyten wird nicht erreicht. Außerdem zeigt sich, dass die in Suspension eingefrorenen Zellen in der Regel innerhalb weniger Stunden nach dem Auftauen ihre metabolische Kompetenz verlieren. Sie sind dann für eine Vielzahl von *in vitro*-Tests ungeeignet.

Weiter sind aus menschlichen Organen isolierte Zellen meist weniger robust als aus Tiermodellen entnommene Zellen. Dies ist vor allem darauf zurückzuführen, dass menschliche Zellen aus Spenderorganen in der Regel unter weniger günstigen Bedingungen gewonnen werden können als Zellen aus Tieren, die meist eigens für diesen Zweck gezüchtet und dann euthanasiert werden (zum Beispiel Ratten, Mäuse oder Schweine). Daher erfordern humane Zellen weitaus schonendere Kultivierungsbedingungen. Bisher ist kein Kryokonservierungsverfahren bekannt, womit humane Hepatocyten erfolgreich kryokonserviert werden können, um damit anschließend über einen längeren Zeitraum *in vitro*-Studien durchführen zu können.

Es besteht daher der Bedarf an verbesserten Verfahren zur Kryokonservierung von Leberzellen, die eine schonende Kryokonservierung, das heißt vor allem ein schonendes Auftauen mit hoher Überlebensrate ermöglichen. Ein verbessertes Kryokonservierungsverfahren soll vor allem geeignet sein, isolierte humane Hepatocyten erfolgreich zu kryokonservieren. Dabei sollte gewährleistet sein, dass die aufgetauten Zellen zu einem großen Anteil, möglichst als konfluenter Monolayer (zusammenhängende Einzelschicht), auf der Kultivierungsmatrix rekultiviert werden können. Weiter soll das Verfahren geeignet sein, verbesserte Sandwich kulturen isolierter Leberzellen herstellen zu können, die einen hohen Anteil vitaler Zellen enthalten. Weiter soll gewährleistet werden, dass die nach der Kryokonservierung aufgetauten, rekultivierten Zellen über einen möglichst langen Zeitraum ihre metabolische beziehungsweise enzymatische Kompetenz aufrechterhalten, sodass sie für eine Vielzahl von *in vitro*-Tests eingesetzt werden können.

Das der vorliegenden Erfindung zugrundeliegende technische Problem besteht demgemäß in der Bereitstellung von verbesserten Verfahren zur Kryokonservierung von isolierten Leberzellen, insbesondere humanen Leberzellen, sodass kryokonservierte Leberzellen schließlich als verbesserte Sandwichkultur rekultiviert werden können.

Das zugrundeliegende technische Problem wird gelöst durch ein Verfahren gemäß Patentanspruch 1, das heißt ein in vitro-Verfahren zur Präparation von Leberzellen für die Kryokonservierung, welches folgende Schritte enthält:

In Schritt (a) wird eine Matrix, insbesondere eine Kollagenmatrix, bevorzugt ein Kollagengel bereitgestellt. Die Matrix ist bevorzugt vorgelegt in einem Zellkulturgefäß, beispielsweise eine 6-well-Platte. Bevorzugt wird das Kulturgefäß mit Matrixmaterial beschichtet.

In Schritt (b) werden isolierte Leberzellen, insbesondere isoliert aus Gewebe, bereitgestellt.

Im nachfolgenden Schritt (c) werden die isolierten Leberzellen auf der Matrix ausgesät. Die Dichte der Leberzellen auf der Matrix beträgt dabei von 2 bis 4 x 10³ Zellen pro mm² Matrixfläche. Die bevorzugte Zelldichte beträgt von 2,6 bis 3,2 x 10³ mm⁻². Das heißt bei einem Kulturgefäß mit einer Grundfläche von 9,6 cm², beispielsweise die Bodenfläche eines Wells in einer 6-well-Platte, beträgt die Zahl der ausgesäten Zellen von 2,5 bis 3 x 10⁶ pro Well.

Im weiteren, vorzugsweise unmittelbar anschließenden, Schritt (d) werden die Zellen auf der Matrix ruhen gelassen (Ruhephase, Anheftungsphase). Dazu wird die mit Zellen belegte Matrix für einen Zeitraum von 10 bis 180 Minuten, bevorzugt von 30 bis 90 Minuten, besonders bevorzugt für etwa 1 Stunde, in Ruhe gelassen, sodass sich die auf der Matrix ausgesäten Zellen an die Matrix anheften können. Das Ruhen erfolgt vorzugsweise im Kulturschrank (Brutschrank), insbesondere bei Standardbedingungen bei einer Temperatur von 37°C, einem Anteil von 5 % CO₂ und bei 95 % relativer Luftfeuchtigkeit. Die erfolgreiche Anhaftung kann durch mikroskopische Kontrolle zusätzlich verifiziert werden.

Nach dem Ruhen in Schritt (d) werden die nicht an der Matrix anhaftenden Zellen in Schritt (e) von der mit Zellen belegten Matrix, insbesondere vorsichtig, abgewaschen (Waschschritt). Das Abwaschen erfolgt vorzugsweise dadurch, dass die mit Zellen belegte Matrix mit Kulturmedium überschichtet wird, und anschließend der flüssige Überstand aus Kulturmedium und nicht anhaftenden Zellen abgesaugt wird. Vorzugsweise wird dieser Schritt mindestens einmal wiederholt.

In einem weiteren Schritt (f) wird die abgewaschene, mit Zellen belegte Matrix erneut ruhen gelassen (zweite Ruhephase). Das Ruhen erfolgt für einen Zeitraum von maximal 180 Minuten, besonders von 30 bis 180 Minuten, besonders bevorzugt für etwa 1 Stunde. Das Ruhen erfolgt vorzugsweise im Kulturschrank, insbesondere bei Standardbedingungen bei einer Temperatur von 37°C, einem Anteil von 5 % CO₂ und bei 95 % relativer Luftfeuchtigkeit.

Nach der zweiten Ruhephase wird die mit Zellen belegte Matrix in Schritt (g) in einem Einfriermedium eingefroren (Einfrierschritt). Vor dem Einfrieren und nach der zweiten Ruhephase wird die mit Zellen belegte Matrix vorzugsweise erneut abgewaschen, vor allem um restliche, nicht anhaftende Zellen zu entfernen (Waschschritt). Das Vorgehen entspricht vorzugsweise Schritt (e).

Das erfindungsgemäße Verfahren sieht also vor, isolierte Leberzellen in einer bestimmten Dichte auf einer Matrix auszusäen und nach einer bestimmten Abfolge von Ruhephasen und Abwaschen nicht anhaftender Zellen in Einfriermedium einzufrieren. Es wird also eine mit Zellen belegte Matrix, insbesondere Kollagenmatrix, worauf die Zellen anhaften und besonders in einem Monolayer vorliegen, eingefroren. Durch die erfindungsgemäßen Maßnahmen wird überraschenderweise eine Kultur intakter Zellen erhalten, die besonders gut eingefroren, das heißt kryokonserviert werden kann. Es stellt sich dabei heraus, dass die erfindungsgemäß präparierten und kryokonservierten Kulturen nach dem Auftauen besonders lebensfähig (vital) sind und eine hohe Zahl von an der Matrix anhaftender Zellen aufweisen. Die aufgetauten Zellen können dabei vorteilhafterweise in einem konfluenten Monolayer rekultiviert werden.

Das erfindungsgemäße Verfahren ist überraschenderweise besonders zellschonend und daher auch für weniger robuste Zellen, wie die aus menschlichen Organen isolierten Hepatocyten geeignet. Vor allem zeigt sich, dass die erfindungsgemäß präparierten und kryokonservierten Zellen, wenn sie nach dem Auftauen mit einer zweiten Matrix überschichtet werden, über mehrere Tage, besonders mehr als 3 Tage, ihre volle metabolische Aktivität beziehungsweise metabolische Kompetenz behalten.

In einer bevorzugten Ausführungsform des erfindungsgemäßen Verfahrens erfolgt das Einfrieren in Schritt (g) mit Einfriermedium, das in einer Menge von etwa 0,5 ml pro mm² Grundfläche des Kulturgefäßes zugegeben wird. Vorzugsweise wird dazu die mit Zellen belegte Matrix mit dem Einfriermedium überschichtet. Vorzugsweise enthält das Einfriermedium 10 % fötales Kälberserum (FCS) sowie 10 % Dimethylsulfoxid (DMSO).

In einer besonders bevorzugten Ausführungsform wird in Schritt (g) nach Zugabe eines Einfriermediums kontrolliert abgekühlt, das heißt eingefroren, vorzugsweise auf Temperaturen von -80°C oder weniger. Vorzugsweise werden zum Abkühlen Kühlraten von -0,5 bis - 20°C pro Minute eingesetzt. In einer bevorzugten Variante wird dabei der Phasenübergang kompensiert; dies erfolgt vorzugsweise durch kurzzeitiges Erwärmen mit Heizraten von vorzugsweise von +1 bis +3°C/Minute.

Nach dem erfindungsgemäßen Präparieren und Einfrieren der isolierten Leberzellen auf einer Matrix wird die mit Zellen belegte Matrix im Rahmen der Kryokonservierung in gefrorenem Zustand gelagert. Vorzugsweise beträgt die Temperatur bei der Lagerung -80°C oder weniger, besonders bevorzugt -150°C oder weniger. Zweckmäßigerweise erfolgt die Lagerung in einem Gefrierschrank oder in der Dampfphase von flüssiger Luft, beziehungsweise flüssigem Stickstoff. Selbstverständlich sind auch alle weiteren bekannten Verfahren zur Tieftemperaturlagerung von Zellen geeignet. Der Fachmann wird die Lagerverfahren entsprechend seinem Anwendungsgebiet und entsprechend ihrer Zweckmäßigkeit wählen.

Die vorliegende Erfindung betrifft demgemäß auch ein Verfahren zur Kryokonservierung isolierter Leberzellen, das die vorstehend charakterisierten Schritte (a) bis (g) enthält, wobei in einem weiteren Schritt (h) die gefrorene, mit Zellen belegte Matrix, über einen unbestimmten Zeitraum, der je nach Anwendungsgebiet und -zweck gewählt wird, gelagert wird.

In einem nachfolgenden weiteren Schritt (i) wird, je nach Anwendungsgebiet und -zweck, vorzugsweise unmittelbar vor der Verwendung oder zu einem anderen geeigneten Zeitpunkt, die gefrorene, mit Zellen belegte Matrix wieder aufgetaut. Dies erfolgt vorzugsweise durch Überschichten der gefrorenen, mit Zellen belegten Matrix mit warmen Kulturmedium. Vorzugsweise liegt die gefrorene, mit Zellen belegte Matrix in einem Kulturgefäß, beispielsweise einer 6-well-Platte, vor. Vorzugsweise wird das Kulturgefäß aus der Gefriertruhe beziehungsweise dem Stickstofftank entnommen und, insbesondere sofort danach, für etwa 5 Minuten im Kulturschrank unter Standardbedingungen (37°C, 5 % CO₂, 95 % relative Luftfeuchtigkeit) inkubiert. Danach wird vorzugsweise ein Auftaumedium (Medium 1), bevorzugt langsam und tropfenweise, auf die mit Zellen belegte Matrix pipettiert. Als Anhaltspunkt werden auf eine Fläche von etwa 9,6 cm² (6-well-Platte) cirka 1 ml Medium 1 gegeben. Die Temperatur des Mediums beträgt vorzugsweise etwa 37°C. Der Vorgang wird für jedes Zellkulturgefäß wiederholt. Als Anhaltspunkt werden maximal drei 6-well-Platten eingesetzt, sodass in maximal 18 Wells die mit Zellen belegte Matrix mit warmen Medium überschichtet wird. Anschließend wird vorzugsweise jedes Zellkulturgefäß beziehungsweise Well mit derselben Menge Medium 1 (1 ml pro 9,6 cm²) in derselben Weise, vorzugsweise langsam und tropfenweise, überschichtet. In einer bevorzugten Ausführungsform ist Medium 1 ein serumhaltiges Auftaumedium, das bevorzugt 10 % fötales Kälberserum (FCS) enthält.

In einem weiteren Verfahrensschritt (j) werden so vor allem nicht anhaftende Zellen oder abgelöste, nicht vitale von der mit Zellen belegten Matrix abgewaschen, sodass eine von nicht anhaftenden Zellen freie Matrix erhalten wird. Dazu wird zunächst der durch die Zugabe von Medium 1 in Schritt (i) erhaltene Überstand über der mit Zellen belegten Matrix möglichst vollständig abgesaugt. Der Überstand enthält im Wesentlichen aufgetaute, nicht vitale und nicht adhärierte Zellen. In einer besonders bevorzugten Ausführungsform wird danach auf die abgesaugte, mit Zellen belegte Matrix ein weiteres Medium, das heißt Medium 2, vorzugsweise tropfenweise, gegeben. Medium 2 hat vorzugsweise eine Temperatur von etwa 37°C. Bei der Zugabe von Medium 2 wird vorzugsweise das in Schritt (i) dargestellte zweischrittige Vorgehen gewählt; zunächst wird die erste Hälfte des Mediums in jeweils alle Zellkulturgefäße verteilt und anschließend die zweite Hälfte entsprechend verteilt. Die zugegebenen Mengen an Medium 2 entsprechen den Mengen an Medium 1 in Schritt (i). Medium 2 hat vorzugsweise eine vom Auftaumedium (Medium 1) abweichende Zusammensetzung. Medium 2 ist vorzugsweise serumhaltig und enthält bevorzugt 10 % FCS.

In einer besonders bevorzugten Ausführungsform werden die mit Medium 2 überschichteten, mit Zellen belegten Matrices danach für etwa 30 Minuten im Kulturschrank unter Standardbedingungen inkubiert (Ruhephase, Inkubation).

Zum Entfernen nicht anhaftender und nicht vitaler Zellen wird danach der Überstand über der mit Zellen belegten Matrix aus dem

Zellkulturgefäß, möglichst vollständig, abgesaugt. Dadurch wird eine im Wesentlichen von nicht anhaftenden und nicht vitalen Zellen befreite mit Zellen belegte Matrix erhalten.

In einem weiteren Schritt (k) wird die aufgetaute, abgewaschene, mit Zellen belegte Matrix mit einer zweiten Matrix, vorzugsweise einer Kollagenmatrix, besonders bevorzugt einem Kollagengel, überschichtet beziehungsweise das Gel aufgegossen. Bevorzugt härtet das vorzugsweise aufgegossene Gel innerhalb weniger Minuten bis 1 Stunde aus. In einer bevorzugten Ausführungsform ist die Zusammensetzung der ersten, unteren Matrix und der nach dem Auftauen aufgebrachten zweiten, oberen Matrix im Wesentlichen gleich, vorzugsweise identisch. Vorzugsweise wird die zweite obere Matrix als Gel auf den auf der unteren Matrix anhaftenden Monolayer aufgetauter Leberzellen aufgegossen. Erfindungsgemäß wird so eine Sandwichkultur erhalten, worin isolierte Leberzellen zwischen zwei Matrices, insbesondere zwei Kollagengelen, eingebettet sind.

In einem abschließenden Schritt (I) werden die zwischen den Matrices eingebetteten Zellen rekultiviert und, je nach Anwendungsgebiet und -zweck, sofort oder nach einer zweckmäßig gewählten Kultivierungsdauer bestimmungsgemäß, das heißt bevorzugt in *in vitro-*Tests verwendet.

Die erfindungsgemäß präparierten und kryokonservierten, aufgetauten und rekultivierten isolierten Leberzellen weisen eine besonders hohe Vitalitätsrate auf. Sie zeigen besonders lange nach dem Auftauen ihre metabolische Kompetenz. Vorteilhafterweise lassen sich durch das erfindungsgemäße Vorgehen vor allem auch aus menschlichen Organen isolierte, weniger robuste Hepatocyten kryokonservieren und erfolgreich auftauen, sodass sie nachfolgend über eine große Zeitspanne in entsprechenden *in vitro*-Tests verwendet werden können. Überraschenderweise wird eine Zellkultur erhalten, worin die Zellen im Wesentlichen als konfluenter Monolayer rekultiviert werden können und worin der Anteil an nicht vitalen beziehungsweise nicht anhaftenden Zellen sehr gering ist.

Die Erfindung betrifft demgemäß auch ein Verfahren zur Herstellung einer Sandwichkultur kryokonservierter isolierter Leberzellen, wobei zumindest die Schritte (a) bis (I) der erfindungsgemäßen Verfahren durchgeführt werden und eine Sandwichkultur von zwischen einer oberen und einer unteren Matrix eingebetteten Zellen erhalten wird.

Gegenstand der vorliegenden Anmeldung ist schließlich auch ein Verfahren zur Herstellung einer Sandwichkultur isolierter Leberzellen, wobei zunächst Lebergewebe eines tierischen oder menschlichen Organismus bereitgestellt wird und anschließend die Leberzellen aus dem Gewebe isoliert werden und zumindest die erfindungsgemäßen Verfahrensschritte (a) bis (I) durchgeführt werden. Der Fachmann wird, je nach Anwendungsgebiet und Zweckmäßigkeit, bekannte Verfahren zur Isolation von Leberzellen aus Geweben wählen.

### Ausführungsbeispiele

Die Erfindung wird in den nachfolgenden Beispielen und Figuren näher erläutert. Die Beispiele sind nicht beschränkend zu verstehen, vielmehr soll damit der der vorliegenden Erfindung zugrundeliegende Erfindungsgedanke näher erläutert und die Vorteile der Erfindung anhand konkreter Beispiele illustriert werden.

Die Figuren zeigen:
- Figur 1:: Mikroskopische Aufnahmen kultivierter Hepatocyten (Maßstab ca. 150fach); Figur 1A: frisch isolierte menschliche Hepatocyten; Figur 1B: kryokonservierte menschliche Hepatocyten;
- Figur 2:: Zahl vitaler Hepatocyten in Abhängigkeit von der Kulti- vierungsdauer;
- Figur 3:: Bildung von 6β -OHT und 16α -OHT nach Enzyminduk- tion mit Rifampicin.

### Beispiel 1: Präparation humaner Hepatocyten für die Kryokonservierung

### 1.1 Isolierung humaner Hepatocyten

Hepatocyten aus menschlichen Spendern wurden in an sich bekannter Weise aus chirurgisch ohnehin entfernten Gewebeteilen, die mit Einverständnis des Spenders entnommen wurden, isoliert. Dazu wurden die Gewebe perfundiert, wobei sich die Hepatocyten aus dem Gewebeverband lösten und aus der Perfusionslösung gewonnen werden konnten. Die Lebensfähigkeit der geernteten Hepatocyten wurde mitteln Trypan Blau-Assay bestimmt. Für die weiteren Experimente wurden nur Zellpräparationen verwendet, die mehr als 70 % Trypan Blau-Ausschluss zeigten.

### 1.2 Bereitstellen einer Matrix

In den folgenden Experimenten wurden Zellkulturgefäße in Form von Multiwellplatten, 6-well-Platten (Typ 657 160, Firma Greiner Bio-One) verwendet. Die Platten wurden mit nativem Kollagengel, das vorzugsweise aus Rattenschwänzen isoliert wurde, beschichtet. Alternativ wurden mit Kollagen Typ 1 vorbeschichtete Multiwellplatten, 6-well-Platten (Typ 657 950 CELLCOAT, Firma Greiner Bio-One) verwendet. Die 6-well-Platten hatten eine Bodenfläche von 9,6 cm² pro Well.

### 1.3 Aussäen der Zellen

Die isolierten Hepatocyten wurden in die Multiwellplatten ausgesät. Dazu wurde eine Suspension der isolierten Hepatocyten hergestellt, welche von 2,5 bis 3 Mio. vitale Hepatocyten pro 2 ml Suspension enthielt. Pro Well der 6-well-Platte wurden 2 ml dieser Zellsuspension einpipettiert. Die Zelldichte betrug somit von 2600 bis 3200 vitale Hepatocyten pro 1 mm² Fläche des Zellkulturgefäßes.

Nach dem Aussäen wurden die Platten für etwa 1 Stunde stehen gelassen. Dazu wurden die Platten in einen Kulturschrank verbracht, worin Standardbedingungen (37°C, 5 % CO₂, 95 % relative Luftfeuchtigkeit) herrschten. Die Ruhephase erlaubte, dass die Zellen aus der Suspension am Kollagengel anhafteten. Die erfolgreiche Anhaftung konnte durch mikroskopische Kontrolle beurteilt werden. Nach der Anheftungs-/Ruhephase wurde der Überstand der Zellsuspension, der im Wesentlichen nicht anhaftende Zellen aufwies, abgesaugt.

### 1.3 Einfrieren der mit Zellen belegten Matrix

Nach dem Absaugen des Überstandes wurde etwa 1 ml 37°C warmes Medium 1 pro Well zugegeben. Danach ruhten die Zellen für etwa eine weitere Stunde erneut im Brutschrank unter Standardbedingungen. Danach wurde der Überstand, der im Wesentlichen nicht anhaftende Zellen enthielt, gründlich abgesaugt.

Medium 1 ist von einem Standardzellkulturmedium für Hepatocyten abgeleitet und enthält 10 % fötales Kälberserum (FCS).

Zum Einfrieren wurden 0,5 ml Einfriermedium pro Well zugegeben. Die Zugabe des Einfriermediums erfolgte zügig. Nach Zugabe des Einfriermediums wurden die Platten sofort in die auf 0°C vorgekühlte Einfriermaschine gestellt und das Einfrierprogramm gestaltet.

Das Einfriermedium basiert im Wesentlichen auf Medium 1 und enhält 10 % fötales Kälberserum (FCS) und 10 % Dimethylsulfoxid (DMSO).

Das Einfrierprogramm sah eine Kompensation des Phasenübergangs vor und erreichte eine Zieltemperatur von -100°C.

Die eingefrorenen Zellkulturplatten wurden anschließend bei -151°C in einer Gefriertruhe beziehungsweise in der Gasphase im Stickstofftank gelagert.

### Beispiele 2: Auftauen kryokonservierter Hepatocyten

Die gemäß Beispiel 1 eingefrorenen und bei -151 °C gelagerten Hepatocytenkulturen wurden für die weitere Verwendung in *in vitro-*Tests nach einer Lagerdauer von bis zu 4 Wochen aufgetaut und rekultiviert.

Zum Auftauen der kryokonservierten Hepatocytenkulturen wurden die 6-well-Platten zunächst unmittelbar nach Entnahme aus dem Gefrierschrank beziehungsweise Stickstofftank für 5 Minuten in einen Kulturschrank verbracht, der mit Standardbedingungen betrieben wurde (siehe Beispiel 1). Anschließend wurde pro Well 1 ml des auf 37°C vorgewärmten Mediums 1 (siehe Beispiel 1) langsam und tropfenweise in jedes Well zugegeben. Dieser Vorgang wurde für maximal drei gleichzeitig aufzutauende 6-well-Platten, das heißt für maximal 18 Wells, wiederholt.

Anschließend wurde der Vorgang wiederholt, wobei erneut pro Well jeweils 1 ml Medium 1 langsam zugefügt wurde. Danach wurde der Überstand mit einer Pasteurpipette abgesaugt. Der Überstand enthielt im Wesentlichen aufgetaute nicht vitale und nicht adhärierte Zellen.

Zum weiteren Abwaschen wurde in gleicher Weise jeweils zweimal 1 ml 37°C warmes Medium 2 zugefügt. Dabei wurde Medium 2 wie vorstehend für Medium 1 beschrieben in zwei Durchgängen à 1 ml in jedes Well zugegeben. Danach wurden die Platten für etwa 30 Minuten im Kulturschrank unter Standardbedingungen inkubiert. Nach der Inkubation wurde der komplette Überstand, der weitere, nicht vitale und nicht adhärierte Zellen enthielt, mit einer Pasteurpipette abgesaugt.

Der so erhaltene Monolayer aus adhärierten Hepatocyten wurde mit einer weiteren Schicht aus Kollagengel überschichtet, um eine Sandwichkonfiguration zu erhalten. Das Kollagengel härtete nach dem Aufgießen nach etwa 30 Minuten aus. Es hatte eine Zusammensetzung, die im Wesentlichen der Zusammensetzung des unteren Kollagengels, das in den Kulturgefäßen vorgelegt war, entsprach.

Medium 2 ist ein Standardmedium für die Langzeitkultivierung von Hepatocyten und enthält 10 % fötales Kälberserum (FCS).

Die weitere Rekultivierung der erhaltenen Sandwichkultur erfolgte im Medium 2, welches etwa alle 24 Stunden durch frisches Medium ersetzt wurde.

### Beispiel 3: Bestimmung der Zahl der lebensfähigen Zellen

Die Zahl der lebensfähigen (vitalen) Zellen wurde in Kulturen frisch isolierter Hepatocyten sowie in Kulturen erfindungsgemäß kryokonservierter Hepatocyten durch Auszählen der morphologisch intakten Zellen bestimmt. Dabei wurden Fotografien von Vergleichsflächen einer Größe von 0,259 mm² ausgezählt. Aus der Zahl der in der Vergleichsfläche aufgefundenen intakten Zellen wurde auf die Zahl intakter Zellen im gesamten Zellkulturgefäß geschlossen (für die verwendeten 6-well-Platten mit einer Fläche von 9,6 cm² pro Well ergab sich ein Korrekturfaktor von 3700).

Vor dem Einfrieren und zu verschiedenen Zeitpunkten nach dem Auftauen nach Kryokonservierung wurde die Morphologie der rekultivierten Hepatocyten fotografisch dokumentiert und mit Kulturen frisch isolierter und kultivierter Hepatocyten verglichen.

### Ergebnisse:

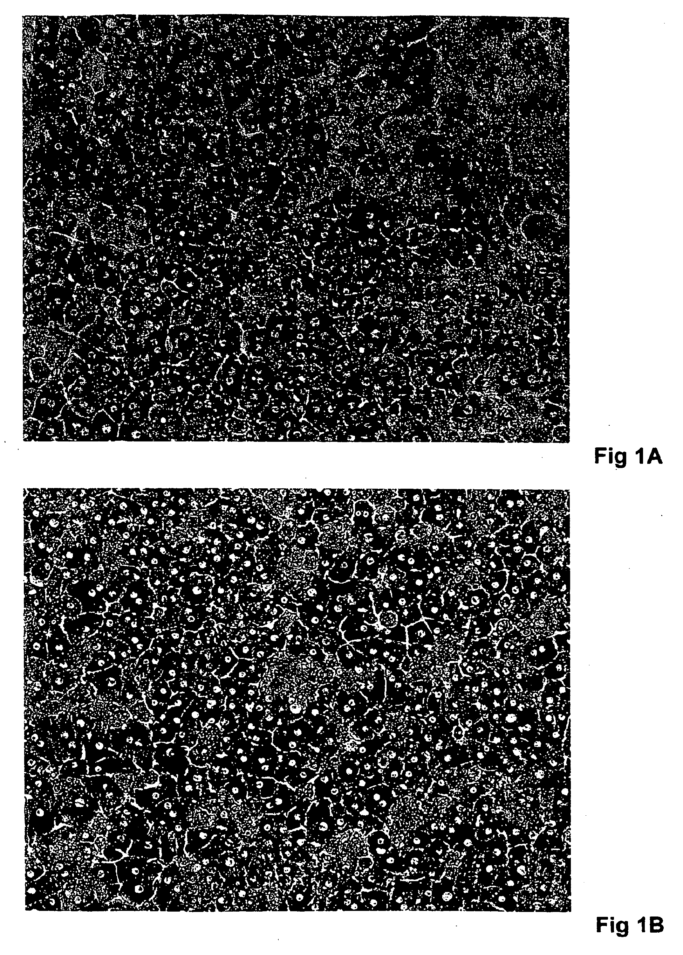

Figur 1 zeigt die Morphologie frisch isolierter und auf einer Kollagengelschicht ausgesäter menschlicher Hepatocyten (Figur 1A) sowie die Morphologie kryokonservierter, aufgetauter und für sieben Tage rekultivierter isolierter menschlicher Hepatocyten (Figur 1 B). Die rekultivierten kryokonservierten Zellen lassen sich morphologisch kaum von frisch isolierten Zellen unterscheiden.

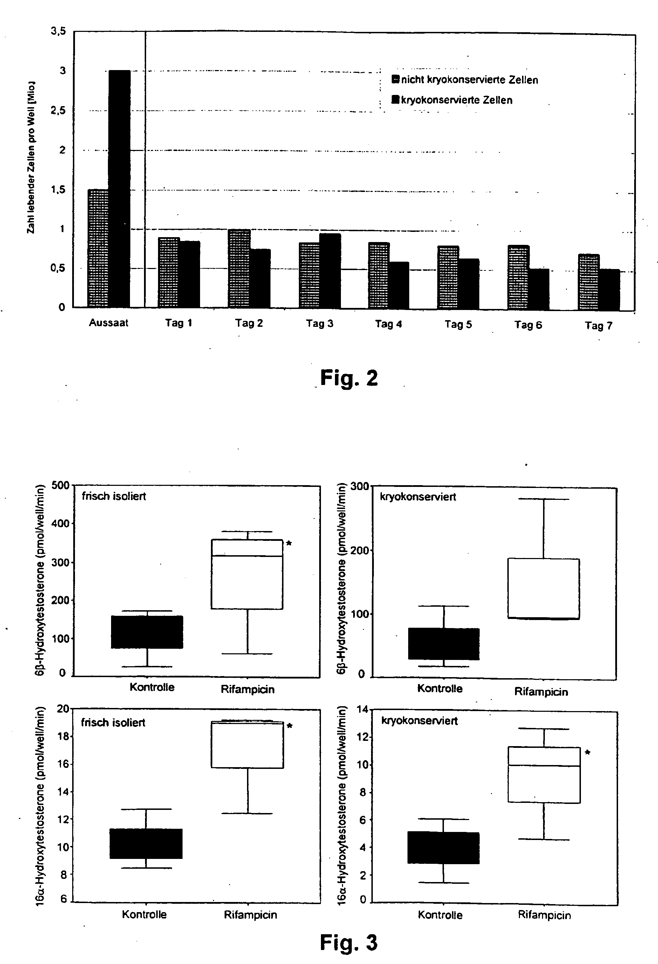

Der Anteil an lebenden Zellen in der Kultur war gegenüber dem Anteil lebender Zellen in Kulturen frisch isolierter Hepatocyten nur gering vermindert. Figur 2 zeigt die Zahl lebender (vitaler) humaner Hepatocyten in Abhängigkeit von der Rekultivierungsdauer nach Auftauen der Hepatocyten. Die Vergleichskurve zeigt die Zahl lebender humaner Hepatocyten bei Kultivierung frischer isolierter Zellen über denselben Zeitraum. Initial wurden für die Kryokonservierung 3 Mio. Zellen, für die Frischpräparation 1,5 Mio. Zellen ausgesät.

Es zeigt sich, dass die aufgetauten rekultivierten humanen Hepatocyten konfluent wachsen und dass sich die Zahl lebender anhaftender Zellen nicht wesentlich von der Zahl lebender Zellen in vergleichbaren Frischkulturen unterscheidet. Bemerkenswert ist dabei, dass auch nach längerer Kultivierungsdauer die Zahl lebender Zellen in den kryokonservierten aufgetauten Präparaten nahezu konstant bleibt.

### Beispiel 4: Enzymaktivität kryokonservierter Hepatocyten

Ein guter Marker für die metabolische beziehungsweise enzymatische Kompetenz von Leberzellen ist die Induzierfähigkeit der enzymatischen Hydroxylierung von Testosteron. In intakten Leberzellen besteht ein Grundumsatz "basal level" dieser Reaktion, dabei wird Hydroxytestosteron (OHT) gebildet. Die Bildungsrate lässt sich bei intakten Zellen durch Enzyminduktion steigern. Der Nachweis der Bildung von OHT in kultivierten Hepatocyten lässt daher Schlüsse auf die enzymatische Kompetenz und physiologische Funktion der kultivierten Hepatocyten zu.

Analyse und Quantifizierung der regio- beziehungsweise stereoselektiven Testosteronhydroxylierung wurde in an sich bekannter Weise, beispielsweise publiziert in Friedrich et al., 2003 (J. Chromatogr. B. 784:49-61), durchgeführt.

Für diese Untersuchung wurden erfindungsgemäß kryokonservierte menschliche Hepatocyten aufgetaut und für zwei Tage rekultiviert und anschließend für weitere 24 Stunden in Rifampicin inkubiert. Rifampicin induziert die Testosteronhydroxylierung an den Positionen 6β, 16α und 2β. Die Testosteronhydroxylierung an Position 6α wird durch Rifampicin nicht stimuliert. Die Messung von 6α hydroxiliertem Testosteron diente daher der Vergleichsmessung der für die Enzyminduktion durch Rifampicin.

Als Kontrolle wurden frisch isolierte Kulturen ebenfalls für 24 Stunden in Rifampicin inkubiert, nachdem sie für zwei Tage kultiviert wurden. Die Kultivierungsbedingungen wurden analog den erfindungsgemäßen Verfahren gewählt.

Neben der Enzyminduzierbarkeit wurde der Testosterongrundumsatz in den kryokonservierten und rekultivierten Zellen sowie in den frisch präparierten und kultivierten Zellen bestimmt.

### Ergebnisse:

Erfindungsgemäß kryokonservierte, rekultivierte Hepatocyten und frisch präparierte kultivierte Hepatocyten zeigten vergleichbare Enzyminduzierbarkeit. Die Mittelwerte für die Induktion der Testosteronhydroxylierung ist in der nachfolgenden Tabelle dargestellt:

| | Kryokonservierte, rekultivierte Hepatocyten (erfindungsgemäß) | Frisch isolierte Hepatocyten (Vergleichsbeispiel) |
|---|---|---|
| 6β-Hydroxylierung | 2,8-fach | 2,3-fach |
| 16α-Hydroxylierung | 2,4-fach | 1,6-fach |
| 2β-Hydroxylierung | 2,3-fach | 2,4-fach |

Wie erwartet, konnte Rifampicin weder in den kryokonservierten und rekultivierten Hepatocyten noch in den frisch kultivierten Hepatocyten die Bildung von 6α -Hydroxytestosteron (6α -OHT) induzieren.

Figur 3 zeigt die absolute Konzentration an gebildetem Hydroxytestosteron (beispielhaft gezeigt für 6β-OHT und 16α-OHT) vor und nach Induktion der Testosteronhydroxylierung mit Rifampicin. Im linken Bildteil sind die Ergebnisse für die frisch kultivierten Hepatocyten, im rechten Bildteil die Ergebnisse für die erfindungsgemäß kryokonservierten und rekultivierten Hepatocyten aufgezeigt. Figur 3A zeigt die Bildung von 6β -OHT, Figur 3B zeigt die Bildung von 16α - OHT. Die Box-and-Whisker-Blots zeigen die Quartilen an. Zeigt das Signifikanzniveau p<0,05 (t-Test) an.

Aus Erfahrungen mit in Suspension kryokonservierten Leberzellen ist bekannt, dass Kryokonservierung den Grundumsatz bei der Testosteronhydroxylierung gegenüber frisch präparierten Heaptocyten stark vermindert. Wie erwartet nahm auch bei den im Monolayer kryokonservierten Leberzellen die Grundaktivität der rekultivierten Hepatozyten im Vergleich zur Frischkultur ab. Sie war jedoch 24 und 72 Stunden nach Auftauen noch gut detektierbar.

Besonders zeigt sich, dass die kryokonservierten Hepatozyten zu einem Zeitpunkt 72 Stunden nach Auftauen und nach vorangegangener 24stündiger Inkubation mit Rifampicin eine deutliche Induzierbarkeit der Testosteronhydroxylierung aufwiesen.

Die über mindestens drei Tage erhaltene Grundaktivität zusammen mit der unverminderten Induzierbarkeit sind wesentliche Hinweise dafür, dass die erfindungsgemäß kryokonservierten menschlichen Hepatozyten erfolgreich für eine Vielzahl von in vitro-Tests eingesetzt werden können.

## Patentansprüche

1. In vitro-Verfahren zur Präparation von Leberzellen für die Kryokonservierung, enthaltend die Schritte:
(a) Bereitstellen einer Matrix,
(b) Bereitstellen isolierter Leberzellen,
(c) Aussäen der Leberzellen auf der Matrix in einer Dichte von 2 bis 4 x10³ mm⁻²,
(d) Ruhen lassen der mit Zellen belegten Matrix für 10 bis 180 min, so dass Zellen an der Matrix anhaften können,
(e) Abwaschen nicht anhaftender Zellen von der mit Zellen belegten Matrix,
(f) Ruhen lassen der mit Zellen belegten Matrix bis maximal 180 min, und
(g) Einfrieren der mit Zellen belegten Matrix in einem Einfriermedium.

2. Verfahren nach Anspruch 1, wobei die Matrix eine Kollagenmatrix ist.

3. Verfahren nach einem der vorhergehenden Ansprüche, wobei in Schritt (d) das Ruhen für 30 bis 90 min erfolgt.

4. Verfahren nach einem der vorhergehenden Ansprüche, wobei in Schritt (e) mit Kulturmedium überschichtet wird und anschließend der flüssige Überstand über der mit Zellen belegten Matrix abgesaugt wird.

5. Verfahren nach einem der vorhergehenden Ansprüche, wobei in Schritt (f) das Ruhen für 30 bis 180 min erfolgt.

6. Verfahren nach einem der vorhergehenden Ansprüche, wobei in Schritt (g) Einfriermedium in einer Menge von 0,5 µl mm⁻² zugegeben wird.

7. Verfahren nach Anspruch 6, wobei das Einfriermedium 10% fötales Kälberserum (FCS) und 10% DMSO enthält

8. Verfahren nach einem der vorhergehenden Ansprüche, wobei in Schritt (g) das Einfrieren erfolgt durch Überschichten der mit Zellen belegten Matrix mit Einfriermedium und kontrolliertes Abkühlen bis - 80°C oder weniger mit Kühlraten von 0,5 bis 20°C min⁻¹, gegebenenfalls mit Kompensation des Phasenübergangs.

9. Verfahren zur Lagerung und Rekultivierung von isolierten Leberzellen, enthaltend die Schritte:
(a) bis (g) des Verfahrens nach einem der Ansprüche 1 bis 8,
(h) Lagern der gefrorenen, mit Zellen belegten Matrix,
(i) Auftauen der gefrorenen, mit Zellen belegten Matrix,
(j) Abwaschen nicht anhaftender Zellen von der mit Zellen belegten Matrix,
(k) Überschichten der aufgetauten, mit Zellen belegten Matrix mit einer zweiten Matrix, und
(l) Rekultivieren der zwischen den Matrices eingebetteten Zellen.

10. Verfahren nach Anspruch 9, wobei in Schritt (h) die Lagerung bei -150°C erfolgt.

11. Verfahren nach Anspruch 9 oder 10, wobei in Schritt (i) die mit Zellen belegte Matrix mit warmem Kulturmedium überschichtet wird.

12. Verfahren nach einem der Ansprüche 9 bis 11, wobei in Schritt (j) die mit Zellen belegte Matrix mit Kulturmedium überschichtet wird und anschließend der flüssige Überstand über der mit Zellen belegten Matrix abgesaugt wird.

13. Verfahren zur Herstellung einer Sandwichkultur isolierter Leberzellen, enthaltend die Schritte (a) bis (I) des Verfahrens nach einem der Ansprüche 9 bis 12.

## Claims

1. In vitro method for the preparation of liver cells for cryopreservation, comprising the steps:
(a) providing a matrix,
(b) providing isolated liver cells,
(c) seeding the liver cells on the matrix in a density of 2 to 4 x 10³ mm⁻²,
(d) allowing the matrix coated with cells to rest for 10 to 180 min, so that cells can adhere to the matrix,
(e) washing off nonadherent cells from the matrix coated with cells,
(f) allowing the matrix coated with cells to rest for up to at most 180 min, and
(g) freezing the matrix coated with cells in a freezing medium.

2. Method as claimed in Claim 1, the matrix being a collagen matrix.

3. Method as claimed in one of the preceding claims, the resting in step (d) taking place for 30 to 90 min.

4. Method as claimed in one of the preceding claims, covering with a layer of culture medium taking place in step (e) and the liquid supernatant above the matrix coated with cells subsequently being aspirated.

5. Method as claimed in one of the preceding claims, the resting in step (f) taking place for 30 to 180 min.

6. Method as claimed in one of the preceding claims, freezing medium in an amount of 0.5 µl mm⁻² being added in step (g).

7. Method as claimed in Claim 6, the freezing medium comprising 10% of fetal calf serum (FCS) and 10% of DMSO.

8. Method as claimed in one of the preceding claims, the freezing in step (g) taking place by covering the matrix coated with cells with freezing medium and controlled cooling to -80°C or less at cooling rates of 0.5 to 20°C min⁻¹, if necessary with compensation of the phase transition.

9. Method for storage and reculturing of isolated liver cells, comprising the steps:
(a) to (g) of the method as claimed in one of Claims 1 to 8,
(h) storing the frozen matrix coated with cells,
(i) thawing the frozen matrix coated with cells,
(j) washing off nonadherent cells from the matrix coated with cells,
(k) coating the thawed matrix coated with cells with a second matrix, and
(l) reculturing the cells embedded between the matrices.

10. Method as claimed in Claim 9, the storage in step (h) taking place at -150°C.

11. Method as claimed in Claim 9 or 10, the matrix coated with cells being covered with warm culture medium in step (i).

12. Method as claimed in one of Claims 9 to 11, the matrix coated with cells being covered with culture medium in step (j) and the liquid supernatant above the matrix coated with cells subsequently being aspirated.

13. Method for the preparation of a sandwich culture of isolated liver cells, comprising the steps (a) to (I) of the method as claimed in one of Claims 9 to 12.

## Revendications

1. Procédé *in vitro* de préparation de cellules hépatiques pour la cryoconservation, comportant les étapes consistant à :
(a) fournir une matrice,
(b) fournir des cellules hépatiques isolées,
(c) semer les cellules hépatiques sur la matrice dans une densité de 2 à 4 x 10³ mm⁻²,
(d) laisser reposer la matrice recouverte de cellules pendant 10 à 180 minutes de sorte que les cellules puissent adhérer à la matrice,
(e) éliminer au lavage les cellules non adhérées de la matrice recouverte de cellules,
(f) laisser reposer la matrice recouverte de cellules jusqu'à 180 minutes au maximum, et
(g) congeler la matrice recouverte de cellules dans un milieu de congélation.

2. Procédé selon la revendication 1, dans lequel la matrice est une matrice de collagène.

3. Procédé selon l'une des revendications précédentes, dans lequel, à l'étape (d), le repos s'effectue pendant 30 à 90 minutes.

4. Procédé selon l'une des revendications précédentes, dans lequel, à l'étape (e), du milieu de culture est appliqué, et le liquide surnageant au niveau de la matrice recouverte de cellules est ensuite aspiré.

5. Procédé selon l'une des revendications précédentes, dans lequel, à l'étape (f), le repos s'effectue pendant 30 à 180 minutes.

6. Procédé selon l'une des revendications précédentes, dans lequel, à l'étape (g), du milieu de congélation est ajouté dans une quantité de 0,5 µl mm⁻².

7. Procédé selon la revendication 6, dans lequel le milieu de congélation contient 10 % de sérum de veau foetal (SVF) et 10 % de DMSO.

8. Procédé selon l'une des revendications précédentes, dans lequel, à l'étape (g), la congélation s'effectue par application de milieu de congélation sur la matrice recouverte de cellules et refroidissement contrôlé jusqu'à -80 °C ou moins avec des vitesses de refroidissement de 0,5 à 20 °C min⁻¹, le cas échéant avec compensation de la transition de phase.

9. Procédé de stockage et de reculture de cellules hépatiques isolées, comportant les étapes consistant à :
(a) à (g) du procédé selon l'une des revendications 1 à 8,
(h) stocker la matrice recouverte de cellules congelée,
(i) décongeler la matrice recouverte de cellules congelée,
(j) éliminer au lavage les cellules non adhérées de la matrice recouverte de cellules,
(k) appliquer une deuxième matrice sur la matrice recouverte de cellules décongelée, et
(l) recultiver les cellules nichées entre les matrices.

10. Procédé selon la revendication 9, dans lequel, à l'étape (h), le stockage s'effectue à -150 °C.

11. Procédé selon la revendication 9 ou 10, dans lequel, à l'étape (i), du milieu de culture chaud est appliqué sur la matrice recouverte de cellules.

12. Procédé selon l'une des revendications 9 à 11, dans lequel, à l'étape (j), du milieu de culture est appliqué sur la matrice recouverte de cellules, et le liquide surnageant au niveau de la matrice recouverte de cellules est ensuite aspiré.

13. Procédé de fabrication d'une culture en sandwich de cellules hépatiques isolées, comprenant les étapes (a) à (I) du procédé selon l'une des revendications 9 à 12.
